# EUROPÄISCHE PATENTSCHRIFT

(11) **EP 1 214 025 B1**
(45) Veröffentlichungstag und Bekanntmachung des Hinweises auf die Patenterteilung: **15.09.2004**
(21) Anmeldenummer: 00960269.9
(22) Anmeldetag: 22.09.2000
(51) Int. Cl.: A61F 2/40

(54) **ENDOPROTHESE FÜR EIN SCHULTERGELENK**
ENDOPROSTHESIS FOR A SHOULDER JOINT
ENDOPROTHESE DE L'EPAULE

(30) Priorität: 24.09.1999 CH 174399; 21.10.1999 DE 29918589 U
(43) Veröffentlichungstag der Anmeldung: 19.06.2002
(73) Patentinhaber: Bähler, André, CH-8032 Zürich (CH)
(72) Erfinder: Bähler, André, CH-8032 Zürich (CH)
(74) Vertreter: Walder, Martin Bernhard
(86) Internationale Anmeldenummer: PCT/CH2000/000515
(87) Internationale Veröffentlichungsnummer: WO 2001/022905

(56) Entgegenhaltungen:
- EP-A- 0 679 375
- EP-A- 0 712 617
- EP-A- 0 715 836
- EP-A- 0 850 609
- EP-A- 0 903 128
- WO-A-98/46172
- DE-A- 19 548 154
- DE-C- 19 512 854
- DE-U- 29 918 589
- FR-A- 2 712 180
- FR-A- 2 773 469

## Beschreibung

### Technisches Gebiet der Erfindung

Die Erfindung betrifft eine Endoprothese für ein Schultergelenk gemäss dem Oberbegriff des Anspruchs 1.

### Stand der Technik

Aus der EP 0 712 617 ist eine Schultergeienkprothese bekannt, deren Schaftteil in seinem metaphysären Ende eine Vertiefung mit hemisphärischem Grund aufweist, in welche Vertiefung eine Kugel eingeführt ist. Diese Kugel besitzt eine zentrale, durchgehende Bohrung und einen mit der Bohrung über deren gesamte Länge verbundenen Schlitz, welcher den Kugelring c-förmig auftrennt. In der Bohrung steckt ein mit der Kalotte verbundener Stiel. Im Schaftteil sind Madenschrauben vorgesehen, um die Kugel in der Vertiefung in einer wählbaren Winkelstellung zu immobilisieren und den Kugelring zu verformen, und dadurch den Stiel darin festzuklemmen.

Nachteilig an dieser Prothese ist, dass die Kalotte bezüglich jeder Richtung genau plaziert sein muss, bevor sie in dieser Stellung fixiert werden kann. Dazu muss sie in dieser Stellung durch einen Chirurgen gehalten werden, währenddem die Madenschrauben kräftig angezogen werden. Zudem kann die optimale Position der Kalotte nur angenähert werden, da der Abstand zwischen der Kalottenachse und der Schaftachse konstant ist. Mit einer Ausrichtung des Kalottenzentrums in Richtung anterior / posterior ist stets auch zwangsläufig eine Verschiebung nach cranial oder caudal verbunden. Überdies müssen die Madenschrauben vor dem Einsetzen der Prothese festgezogen werden, da sie danach nicht mehr zugänglich sind. Dies bedingt während der Operation mehrfache Probemanipulationen am operierten Arm und Korrekturen an der Knochenschnittfläche, und verunmöglicht eine Korrektur der Ausrichtung nach erfolgter Implantation.

Die FR 2 773 469 offenbart eine Schultergelenkprothese mit Schaftteil, daran einem verschwenkbaren Zwischenstück oder Richtstück, welches Zwischenstück eine Kugelkalotte trägt. Bei dieser Prothese ist am metaphysären Schaftteilende eine hemisphärische Grube mit einer Gewindebohrung an deren Grund vorgesehen. Grube und Gewindebohrung sind auf eine Halsachse ausgerichtet, deren Winkel zur Schaftachse vorgewählt ist. In der Grube sitzt ein verschwenkbares Zwischenstück mit einer zur Halbkugeloberfläche der Grube konzentrischen Halbkugeloberfläche an seinem humeralen Ende. Das Zwischenstück besitzt am anderen Ende eine bezüglich des beiden Kugeloberflächen gemeinsamen Kugelmittelpunkts exzentrische Konusoberfläche, auf welcher die Kopfkalotte mit einer entsprechenden konischen Vertiefung sitzt. Die Achse dieser Vertiefung ist bezüglich dem Kalottenmittelpunkt exzentrisch angeordnet. Das Zwischenstück besitzt eine axiale Bohrung mit einem halbkugelförmigen Grund. Eine Schraube mit Kugelkopf ist mit dem Schaft voran in die Bohrung eingeführt, wobei der Gewindeabschnitt des Schaftes durch eine konische Bohrung im halbkugelförmigen Grund des Zwischenstückes hindurchgestossen und in die Gewindebohrung im Schaftteil eingeschraubt ist. Die Zentren der Kugeloberflächen von Grube im Schaftteil, innerer und äusserer Halbkugeloberfläche am Zwischenstück und Kugeloberfläche des Schraubenkopfes liegen bei angezogener Schraube in einem gemeinsamen Zentralpunkt. Dadurch ist das Zwischenstück gegenüber dem Schafteil in alle Richtungen verschwenkbar und gleichzeitig verdrehbar. Durch eine Verschwenkung des Zwischenstückes wird dessen Längsachse parallel zur gewünschten Halsachsenrichtung gestellt, und durch ein Verdrehen des Zwischenstückes um seine Längsachse und ein auf diese Verdrehung abgestimmtes Verdrehen der Kalotte kann die Abweichung der zu erzielenden Halsachse von der angenäherten Halsachse ausgeglichen werden. Steht die optimale Lage des Zwischenstückes fest, kann es durch Festziehen der Schraube am Schaftteil fixiert werden.

Auch bei dieser Prothese ist die Richtung des Gelenkhalses stufenlos gemäss den Gegebenheiten des ursprünglichen Gelenkes einstellbar. Durch Einstellen von Inklination und Rotation bezüglich der Schaftachse mit Hilfe eines Manipulierkopfes ist die Halsachse parallel zur optimalen Halsachse einstellbar. Diese Annäherung ist danach quer zur Achsenrichtung mit den zusammenwirkenden exzentrisch angeordneten Konusoberflächen an Zwischenstück und Kalotte stufenlos korrigierbar. Die Länge des Gelenkhalses wird durch ein Set von Kalotten unterschiedlicher Dicke reguliert. Dadurch lässt sich eine Kopfkalotte an jedem beliebigen Ort in jeder gewünschten Stellung festmachen, so dass der Ort der Kalotte optimal auf die ursprünglichen Verhältnisse des Gelenkes oder die beabsichtigte Korrektur ausgerichtet und das ursprüngliche Drehzentrum des Schultergelenkes wiederhergestellt werden kann.

Da der Erfolg einer Schulterprothese wesentlich von der Wiederherstellung des muskulären Gleichgewichts der Schultermuskulatur abhängt, ist es wichtig, dass das ursprüngliche Drehzentrum des Schultergelenkes durch diese individuelle Plazierbarkeit der Kopfkalotte wiederherstellbar ist. Auch ist ein möglichst geringer Abstand zwischen Knochenschnittfläche und künstlicher Kalotte erzielbar, so dass den Muskeln bei jeder Bewegung eine kontinuierliche Auflage geboten wird. Vorteilhaft ist auch, dass Inklination und Rotation des Zwischenteils bezüglich des Schaftteils auch nach der Implantation des Schafteils auf die Knochenschnittfläche bzw. die gewünschte Ausrichtung der Kalotte ausgerichtet und in dieser Stellung fixiert werden können, da die das Zwischenteil fixierende Schraube von der Kalottenseite her zugänglich bleibt.

Nachteilig an dieser Prothese ist jedoch, dass die Kugeloberflächen exakt konzentrisch hergestellt werden müssen, was aufwändig und kostspielig ist. Kugelgelenke bieten zudem eine sehr geringe Auflagefläche. Diese Auflagefläche ist überdies im Bereich der Hauptzugrichtung der Schraube durch die konische Bohrung in der Kugelwandung des Zwischenstückes verkleinert. Kugelgelenke weisen generell den Nachteil auf, dass sie keinen Pressfit im Sinne einer konischen Verklemmung zulassen. Die Fixierung dieses Kugelgelenkes bleibt daher auch nach Abschluss der Operation unsicher.

Ausserdem muss das Zwischenstück in jeder Beziehung richtig platziert sein, bevor es fixiert werden darf. Insbesondere ist ein Drehen um die Richtachse, nämlich eigene Längsachse, um den exzentrischen Konus in die gewünschte Stellung zu drehen, nur möglich, wenn auch die Winkellage des Zwischenstückes instabil ist. Eine unabsichtliche Verstellung des Zwischenstückes ist beim Abnehmen des Manipulierkopfes, beim Einsetzen der Schraube, beim Anziehen der Schraube und beim Einstellen und Aufsetzen der Kopfkalotte möglich.

Die richtige Drehstellung des Zwischenstückes zu finden, bei welcher die beiden exzentrischen Konusoberflächen an Zwischenstück und Kopfkalotte derart zusammenspielen, dass die Kopfkalotte an die gewünschte Stelle zu liegen kommt, ist sehr schwierig. In der Praxis wird daher die Exzentrizität der Konusoberfläche am Zwischenstück mit Hilfe eines Manipulierkopfes derart gerichtet, dass die Konusachse möglichst nahe der als optimal geschätzten Achse liegt und danach die Kalotte aufgesetzt. Mit der Ausrichtung des Kalottenzentrums in Richtung anterior / posterior ist dann aber stets zwangsläufig auch eine Verschiebung nach cranial oder caudal verbunden.

### Aufgabe der Erfindung

Es ist daher Aufgabe der Erfindung, eine Endoprothese für ein Schultergelenk vorzuschlagen, bei welcher die Vorteile des zitierten Standes der Technik beibehalten und die erwähnten Nachteile von Kugelgelenken vermieden werden. Es sollen insbesondere möglichst grosse Reibungskräfte zwischen den einzelnen, gegeneinander verschiebbaren und verdrehbaren Teilen erreicht werden. Ferner soll es möglich sein, die Richtung und die Lage der Gelenkachse unabhängig voneinander festlegen zu können. Die Justierung von Richtung und Lage der Kalottenachse soll gegenüber dem Stand der Technik vereinfacht werden. Die Empfindlichkeit der Einstellungen der Achsenrichtung bezüglich Inklination und Rotation auf unbeabsichtigte Veränderungen der Inklinations- oder Rotationsstellung durch Verstellen der anderen Einstellung soll möglichst minimiert werden. Auch soll die Richtung der Achse durch Veränderung der Lage der Kalottenachse in Bezug auf die Lage der Längsachse des Zwischenstücks oder Richtstückes möglichst unbeeinflussbar sein.

### Beschreibung der Erfindung

Diese Aufgabe wird durch die Prothese gemäß Anspruch 1 gelöst.

Durch diese Trennung der Schwenkbewegung des Richtstückes in zwei Schwenkbewegungen um zwei sich querende Achsen bleibt der gesamte Bewegungsspielraum, den ein Kugelgelenk bietet, erhalten, doch ist im Vergleich zu einem Kugelgelenk die Gefahr der unbeabsichtigten Veränderung einer Schwenklage bezüglich der einen Achse durch eine Verschwenkung um die andere Achse deutlich verkleinert. Die Anlenk- und Reibungsflächen zwischen Schaftteil, Drehstück und Richtstück sind keine Kugeloberflächen, sondern Oberflächen von axialen Rotationskörpern, z.B. Zylinder, Torus, Konus, um nur die einfachsten zu nennen, deren Oberflächen einfacher präzise herstellbar sind. Die Berührungsflächen können daher grossflächiger als Kugeloberflächen ausgebildet sein. Auch ist ein Pressfit möglich, indem konische Abschnitte in der Anlenkfläche vorgesehen werden können. Dabei ist es neben der zur Halsachse parallelen Ausrichtung der ersten Achse auch möglich, die erste Achse etwa senkrecht zur Halsachse oder zur Richtachse und die zweite Achse wiederum etwa senkrecht zu diesen beiden zu wählen. Ferner ist es auch möglich die erste Achse mit einer Richtungsabweichung von der Richtung der Halsachse auszurichten. Diese Abweichung beträgt vorteilhaft weniger als 60 Grad, vorzugsweise weniger als 50 Grad. Aus dann wird die zweite Achse die erste querend angelegt. In der Folge kann sowohl die Rotationsstellung als auch die Inklinationsstellung eingestellt werden. Vorteilhaft wird die Richtung der ersten Achse etwa parallel zu oder besser in einer Ebene durch Schaftachse und Halsachse gewählt. Bei einer Verschwenkung oder Rotation eines Prothesenteils um eine Achse, die parallel zu oder in dieser Ebene liegt, wird die Inklination nicht verstellt. Es wird lediglich die Ausrichtung der die erste Achse querenden zweiten Achse verstellt. Dadurch ist die durch die zweite Achse gegebene Verstellrichtung einstellbar. Insbesondere ist eine zur Halsachse parallele Ausrichtung der ersten Achse zweckmässig.

Schneidet die erste Achse die Halsachse, oder fallen beide zusammen, so bewirkt eine Verschwenkung des Drehstücks um diese erste Achse praktisch keine Verlängerung oder Verkürzung des Gelenkhalses.

Vorteilhaft ist wenigstens ein Exzenterring oder ein Langlochring zwischen Richtstück und Kopfkalotte vorgesehen. Dieser Ring erlaubt die Lage der Kalotte, bzw. der Abstand zwischen der Richtachse und dem Zentrum der Kalotte zu verändern und festzulegen. Dies erlaubt das Wählen von Richtung und Betrag des Abstandes zwischen der Richtachse und der Kalottenachse bzw. dem Zentrum der Kopfkalotte. Auf diese Art kann auch eine aus einer Verschwenkung um die erste Achse resultierende Verschiebung der zweiten Achse ausgeglichen werden. Deshalb müssen sich die erste und die zweite Achse nicht in einem Punkt schneiden.

In einer vereinfachten Ausführungsform kann die Kalotte direkt auf das Richtstück aufgesetzt sein. Dabei kann die Vertiefung, mit der sie auf das Richtstück gesetzt wird zentrisch oder exzentrisch in der Kalotte eingearbeitet sein. Die Kalotte braucht auch nicht eine Kugeloberfläche aufzuweisen, sondern kann eine von dieser idealisierten Artikulationsfläche abweichende Ausbildung aufweisen.

Zweckmässigerweise weist das Richtstück eine äussere Zylinder- oder Konusoberfläche auf, deren Rotationszentrum die Längsachse des Richtstückes, d.h. die Richtachse ist. Auf diese Oberfläche ist die Kopfkalotte, der Exzenterring bzw. der Langlochring aufsetzbar. Vorgezogen wird die Konusoberfläche, auf welcher diese Teile durch Aufpressen am Richtstück fixiert werden können. Für den Langlochring, der ohnehin eine Feststelleinrichtung, z.B. Madenschrauben, benötigt, kann die Oberfläche gut zylindrisch sein. Zylinderoberflächen in Verbindung mit Feststelleinrichtungen, sowohl beim Langlochring wie bei einem Exzenterring, erlauben ein Wählen des Abstandes zwischen diesem Ring und dem Schaftkopf, und demzufolge des Abstandes zwischen dem Kopfkalottenmittelpunkt der darauf angeordneten Kalotte einerseits und der Schaftachse andererseits.

Vorzugsweise entspricht die Ausrichtung der ersten Achse der Ausrichtung einer vorgewählten Halsachse. Dadurch ist das Drehstück um die Halsachse drehbar im Schaftteil gelagert und kann am Schaftteil mit sehr einfachen Mitteln drehfest befestigt werden. Durch Verstellen des Richtstückes um die zweite Achse gegenüber dem Drehstück sind, zumindest in einem engeren Bereich um die mittlere Stellung herum, kaum Kräfte zu erwarten, welche das Drehstück um die erste Achse zu schwenken vermögen, da diese beiden Achsen bzw. Schwenkrichtungen im Wesentlichen senkrecht zueinander stehen. Da die erste Achse in etwa parallel zur Richtachse verläuft, ist zur Rotation um diese erste Achse eine grundsätzlich andere Manipulation notwendig als zum Schwenken um die senkrecht zur Richtachse stehende zweite Achse.

Vorteilhaft weist das Drehstück eine gegen das Richtstück gerichtete konkave Rotationskörperoberfläche mit der zweiten Achse als Rotationszentrum auf. Alternativ weist das Drehstück jedoch eine gegen das Richtstück gerichtete konvexe Rotationskörperoberfläche mit der zweiten Achse als Rotationszentrum auf. Insbesondere sind eine Zylinder-, Torus- oder Konusoberfläche, aber auch kompliziertere Rotationskörperoberflächen, z.B. mit steilen konischen Seitenflächen oder mehrdirektional gewölbten Flächen denkbar, ebenso eigentliche Scharniergelenke mit eingelegten Achsenbolzen.

Wenn auch die beiden Achsen sich nicht schneiden müssen, so schneidet die erste Achse die zweite Achse in einer Ausführungform in einem Zentralpunkt. Dadurch ist es möglich, die beiden Schwenkbewegungen mit einer gemeinsamen Feststelleinrichtung gemeinsam zu fixieren. Diese Feststelleinrichtung, welche vorzugsweise eine Schraube ist, die in eine Gewindebohrung im Schaftteil eingeschraubt werden kann, und das Richtstück müssen dazu Berührungsflächen aufweisen, welche auf Kugeloberflächen liegen, deren Zentren in verschraubtem Zustand mit dem Schnittpunkt der beiden Achsen, dem Zentralpunkt, zusammenfallen.

Vorteilhaft liegt im Schaftteil eine Ausnehmung mit einer Seitenwandung vor, welche Seitenwandung einer Rotationsfläche um die erste Achse oder die Halsachse entspricht. Darin ist ein Drehstück mit einem der Seitenwandung entsprechendem Umriss um die erste Achse drehbar gelagert, welches Drehstück eine Anlenkfläche für das Richtstück in Form einer Rotationsfläche um die zweite Achse aufweist. Die Seitenwandung kann zylindrisch, konisch oder mit einem Gewinde versehen sein. In jedem Fall kann das Drehstück vor der Fixierung um mind. 360° gedreht werden. Bei der Fixierung ergibt sich im Falle der konischen Seitenwandung eine konische Verklemmung an der Seitenwandung, bei der zylindrischen Seitenwandung erfolgt die Verpressung über die Bodenfläche der Vertiefung bzw. des Drehstücks. Auch bei einem Gewinde erfolgt die Verpressung über grosse Flächen, nämlich die Gewindeflanken.

Das Richtstück weist eine der richtstückseitigen Anlenkfläche des Drehstücks entsprechende Gegenform und eine axiale Konusoberfläche zum Aufstecken einer Konusoberfläche der Kopfkalotte oder eines Exzenterringes auf. Die Anlenkflächen zwischen Richtstück und Drehstück müssen sich nicht flächig entsprechen, sondern können auch lediglich linear oder gar punktuell aufliegen. Zur Befestigung des Richtstücks am Schaftteil ist vorteilhaft im Schaftteil eine Gewindebohrung vorgesehen. Das Richtstück weist eine Innenbohrung, in welcher der Kopf einer Schraube Platz findet, sowie eine Bohrung für den Schraubenschaft auf. Mit der Schraube ist das Richtstück durch eine Bohrung im Drehstück hindurch in der Gewindebohrung festschraubbar. Die Bohrung im Richtstück für den Schraubenschaft ist als Schlitz ausgebildet, welcher einen Bewegungsbereich des Schraubenschaftes von ± 30° zulässt und dessen Breite dem Durchmesser eines mit der Bohrung zusammenwirkenden gewindelosen Teils des Schraubenschaftes angepasst ist, um ein seitliches Verschieben des Richtstücks gegenüber dem Drehstück zu verhindern.

In einer anderen Ausführungsform sind die beiden unabhängigen Drehbewegungen um die erste und die zweite Achse auch einzeln fixierbar. Dies erleichtert das Feststellen oder Fixieren der einzelnen Schwenkbewegungen. Dadurch kann z.B. zuerst die Rotation der Richtachse bezüglich der Schaftachse festgelegt und fixiert werden, um danach in einem zweiten Schritt die Inklination zu bestimmen und zu fixieren.

Wo es die Verhältnisse zulassen, kann die Kopfkalotte an Stelle einer zentrisch angeordneten eine bezüglich der Kopfkalotte exzentrisch angeordnete innere Konusoberfläche aufweisen, welche auf die äussere Konusoberfläche des Richtstücks oder des Exzenterringes aufsteckbar ist. Dadurch kann der Abstand und die Richtung zwischen Kalottenzentrum und Längsachse des Richtstückes eingestellt werden. Die Exzentrizität sowohl der inneren Konusoberfläche in der Kalotte als auch der äusseren Konusoberfläche am Exzenterring ist dabei aber nicht sichtbar, sondern durch die Kalotte verdeckt. Dadurch ist das gezielte Einstellen von Richtung und Distanz erschwert. Deshalb sind vorteilhaft zwei über Konusoberflächen zusammensteckbare Exzenterringe zwischen Kopfkalotte und Richtstück angeordnet. Diese erlauben die Sichtkontolle der Lage der Konusumrisse zueinander und in Bezug zum Umriss der Knochenschnittfläche, so dass die Exzenterringe ins Zentrum der Umrisslinie eingemittet werden können, um nachher eine Kalotte mit zentrischer Konusoberfläche auf den zweiten Exzenterring aufzusetzen.

Vorteilhaft wird jedoch anstelle eines zweiten Exzenterringes ein Manipulierkopf verwendet. Dieser weist eine bezüglich der Kalottenachse exzentrisch angeordnete innere Konusoberfläche auf, welche auf die äussere Konusoberfläche des Exzenterringes aufsteckbar ist und deren Exzentrizität der Exzentrizität der inneren Konusoberfläche der Kopfkalotte entspricht. Zudem ist er derart ringförmig ausgestaltet, dass der Exzenterring durch die exzentrische Ringöffnung des Manipulierkopfes hindurch sichtbar und verstellbar ist. Dies erlaubt beim Einstellen des Exzenterringes den optischen Vergleich zwischen dem Rand des Manipulierkopfes und dem Rand der Knochenschnittebene. Weil die Exzentrizitäten von Kalotte und Manipulierkopf übereinstimmen, kann die optimale Drehstellung des Exzenterringes durch diesen Vergleich leicht festgelegt werden, der Exzenterring am Richtstück festgeschlagen oder festgepresst werden und die Kalotte in der gleichen Ausrichtung wie der Manipulierkopf an dessen Stelle gesetzt werden. Das Auffinden der optimalen Drehstellung der Kalotte bei optimal gerichtetem Richtstück und Exzenterring ist danach leicht.

Alternativ zu einem Exzenterring zwischen Richtstück und Kalotte ist es auch möglich ein Exzenterstück zwischen Drehstück und Schaftteil vorzusehen. Die Fixation des Exzenterstück am Schaftteil kann z.B. mit einer Ringschraube geschehen. Drehstück und Richtstück müssten dann am Exzenterstück befestigt werden.

Alternativ zum Doppelten Exzenterring kann ein Langlochring vorgesehen sein, welcher ein Langloch aufweist, mit welchem er auf das Richtstück aufsetzbar ist. Der Ring weist eine Feststelleinrichtung auf, mit welcher er in einer wählbaren Verschiebe- und Drehstellung bezüglich dem Richtstück an diesem fixierbar ist. Die Verwendung eines Langlochringes zwischen Richtstück und Kopfkalotte ist unabhängig von der schaftteilseitigen Verschwenkeinrichtung. Ein Langlochring kann dementsprechend auch bei einer Prothese gemäss dem bekannten Stand der Technik eingesetzt werden.

Bei einer für die Produktion vorteilhaften Ausführung des Richtstücks liegt dessen Ausdehnung innerhalb eines Walzen- oder Zylinderstücks mit dem Radius der konvexen Anlenkfläche. Dies ermöglicht es, das Richtstück aus einem Walzenstück mit diesem Radius herzustellen, indem durch spanabhebende Mittel das Verbindungsteil zum Gelenkkopf mit der Innenbohrung aus dem Walzenstück herausgearbeitet wird.

Bei einer zur obigen, mit einer im Schaftteil eingeschraubten Schraube alternativen Ausführung ist im Drehstück eine mit der Anlenkfläche zwischen Drehstück und Richtstück konzentrische Bohrung vorgesehen und in der Bohrung ein zylinderförmiger Körper angeordnet. Dieser Körper oder Kopfteil ist mit einem Schraubenschaft verbunden, wobei die Wandung zwischen Bohrung und Anlenkfläche einen quer zur zweiten Achse verlaufenden Schlitz aufweist, durch welchen der Schraubenschaft hindurchsteht. Das Richtstück weist eine Anlenkscheibe und eine Druckscheibe auf, wobei die Anlenkscheibe eine Bohrung für den Schraubenschaft und eine der Anlenkfläche des Drehstücks entsprechende Anlenkfläche, sowie auf der dieser gegenüberliegenden Seite eine mit der Druckscheibe zusammenwirkende Druckfläche aufweist. Die Druckscheibe steht mit dem Schraubenschaft in Verbindung. Die Verbindung zwischen Druckscheibe und Schraubenschaft gewährt, dass durch Drehen des Druckkörpers oder des Schraubenschaftes eine relative Verdrehung zwischen Schraubenschaft und zylindrischem Körper und/oder Druckkörper resultiert und dadurch die Distanz zwischen zylindrischem Körper und Druckkörper verändert wird. Dafür ist entweder im zylindrischen Körper oder im Druckkörper, oder in beiden, ein Innengewinde vorgesehen, welches mit einem Aussengewinde am Schraubenschaft zusammenwirkt. Dadurch ist das Richtstück und das Drehstück aneinander fixierbar. Deshalb ist das Drehstück seinerseits unabhängig von der Fixierung des Richtstückes am Drehstück vorteilhaft mit einem auf oder in das Schaftteil schraubbaren Ring am Schaftteil fixierbar.

Vorteilhaft sind bei einer solchen Endoprothese für ein Schultergelenk wenigstens zwei der folgenden Teile vor der Implantation der Prothese zusammengesetzt und wenigstens provisorisch aneinander befestigt: Schaftteil, Drehstück inklusive allfälligem Kopfstück, Richtstück inkl. allfälliger Anlenk- und Druckscheibe, und Schraube, sowie allfälliger Exzenterscheibe oder Langlochscheibe. Diese Teile sind z.B. mit Überwurfringen oder Zungen vor dem Auseinanderfallen gesichert. Dies hat den Vorteil, dass der Chirurg nicht schlecht handhabbare Kleinteile in die Finger nehmen muss. Eine zweckmässige Zusammenstellung aus der Reihe der Möglichkeiten ist, das Schaftteil mit dem daran gesichertem Drehstück und gesondert das Richtstück, allenfalls zusammengesetzt aus Anlenk- und Druckscheibe, mit eingesetzter Schraube oder Schraubenschaft vorzusehen. Das Drehstück kann auch mit dem Richtstück zusammengefasst oder alle Teile provisorisch zusammengesetzt sein.

Die zusammenwirkenden konkaven und konvexen Berührungsflächen zwischen Drehstück und Richtstück, zwischen Richtstück und Schraube, bzw. zwischen Richtstück und Anlenkscheibe können einander in Geometrie und Grösse entsprechen. Sie können sich aber auch lediglich derart entsprechen, dass eine Linienberührung zwischen den Berührungsflächen stattfindet. Dazu ist beispielsweise die Anlenkfläche am Drehstück als Rinne mit zwei in einem Winkel zueinander stehenden ebenen Flächen ausgebildet. Die Bohrung im Richtstück, in welche die Schraube eingesetzt ist besitzt entsprechend nicht eine Halbkugelhöhlung, sondern eine konische Bohrung, oder eine oder mehrere Kreiskanten entlang etwa derselben geometrischen Kugeloberfläche. Die Anlenkscheibe wiederum ist dem Drehstück entsprechend mit z.B. zwei winklig zueinander stehenden, ebenen Flächen ausgerüstet. Die Berührungsflächen können auch beim Festziehen der Schraube in die Gegenfläche einpressbare Kanten oder Dorne aufweisen. Die Linienberührung hat den Vorteil gegenüber der Flächenberührung, dass die Berührungsflächen unter Materialverformung aneinander gepresste werden und die Verbindung bei angezogener Schraube dadurch sehr stabil ist. Die Ausgestaltung der konkaven Berührungsflächen in einer Art, dass sie die konvexe Berührungsfläche lediglich in einer Linie berühren, hat zudem den Vorteil, dass die konkaven Berührungsflächen sehr einfach und kostengünstig herstellbar sind.

### Kurze Beschreibung der Figuren

Im Folgenden wird die Erfindung anhand von in den Figuren schematisch dargestellten Beispielen näher erläutert. Es zeigt:
- Figur 1:: eine Explosionsdarstellung mit z.T. geschnittenen Teilen eines ersten Ausführungsbeispiels,
- Figur 1a:: zwei Varianten der Schraube 53 gem. Figur 1,
- Figur 1b:: eine Variante des Richtstücks 41 gemäss Figur 1,
- Figur 2:: eine Aufsicht auf einen Langlochring,
- Figur 3:: eine Aufsicht auf einen Exzenterring,
- Figur 4:: eine Untersicht und eine Seitenansicht des Richtstücks gem. dem in Fig. 1 dargestellten Beispiel,
- Figur 5:: eine Seitenansicht und eine Aufsicht auf das Drehstück gem. dem in Fig. 1 dargestellten Beispiel,
- Figur 6:: einen Schemaschnitt durch ein zweites Ausführungsbeispiel in zusammengesetztem Zustand,
- Figur 7:: einen Schemaschnitt durch ein drittes Ausführungsbeispiel, mit Andeutung des Humerus, mit ebenflächigen Berührungsflächen an der Anlenkscheibe.

### Beschreibung der Ausführungsbeispiele

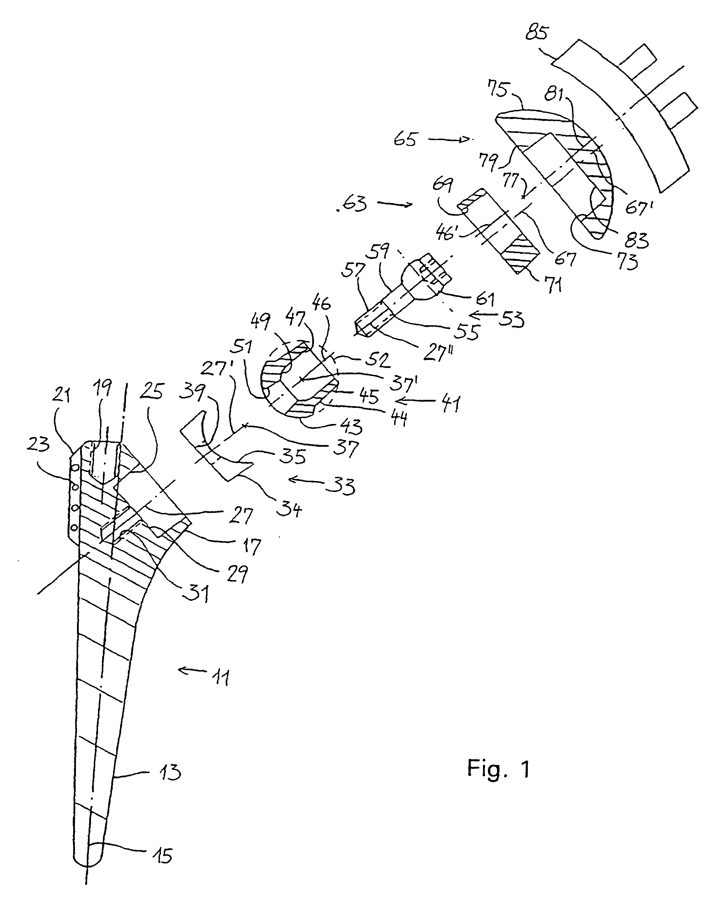

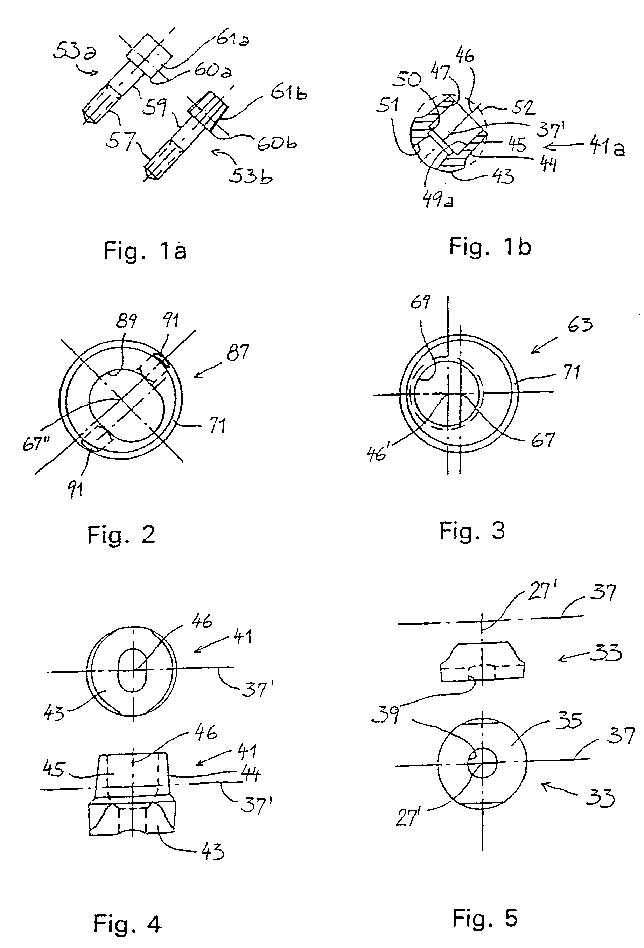

Das erste Ausführungsbeispiel gemäss Figuren 1 bis 5 weist einen Schaftteil 11 mit einem Schaftstiel 13 entlang einer Schaftachse 15 und einem Schaftkopf 17 auf. Am Schaftkopf 17 ist eine Vorrichtung 19 zum Einbringen des Schaftstiels 13 in den Knochen vorgesehen. Diese dient auch zum Entfernen desselben aus dem Knochen. Ferner ist an Schaftkopf 17 eine Rippe 21 mit Bohrungen 23 vorgesehen. An dieser Rippe 21, bzw. an den Bohrungen 23 können Weichteile angeheftet werden. Weiter ist am Schaftkopf 17 eine konkave, kegelstumpfförmige Anlenkfläche 25 vorgesehen, deren Achse 27 parallel zur Halsachse hier in einem Winkel von 135° zur Schaftachse 15 steht und im Folgenden erste Achse genannt wird. Axial zu dieser ersten Achse 27 ist im Boden 29 der Anlenkfläche 25 eine Bohrung 31 mit Innengewinde vorgesehen.

In die konkave Vertiefung der Anlenkfläche 25 am Schaftteil 11 ist ein Drehstück 33 einsetzbar (siehe auch Figur 5). Das Drehstück 33 ist eine Konusscheibe, bzw. ein Kegelstumpf, mit einer Neigung des Kegelmantels 34, welche der Kegelmantelneigung der Anlenkfläche 25 entspricht. Auf der Seite der Kegelstumpf-Grundfläche weist das Drehstück 33 eine zylindrische Ausnehmung 35 mit einer zweiten Achse 37 quer ersten Achse 27 auf. Axial zur ersten Achse 27 ist im Drehstück 33 eine Bohrung 39 vorgesehen.

In die Ausnehmung 35 passt das Richtstück 41 (siehe auch Figur 4). Dieses weist eine zylindrische Anlenkfläche 43 auf, welche mit der Zylinderoberfläche 35 der Ausnehmung übereinstimmend gewölbt ist. Die Achsen der einander entsprechenden Oberflächen decken sich in zusammengebauten Zustand, weshalb die Achse der Anlenkfläche 43 am Richtstück 41 mit der Bezugsziffer 37' bezeichnet ist. Ferner ist am Richtstück 41 ein Verbindungsteil 44 mit einer Kegelstumpfmantel- bzw. Konusfläche 45 vorgesehen. Die Achse 46 des Verbindungsteils 44 und der Konusfläche 45 steht senkrecht zur Achse 37' der Anlenkfläche 43. Auf diesen Verbindungsteil 44 ist von der von der Anlenkfläche 43 abgewandten Seite her der Gelenkkopf der Prothese aufsetzbar. Das Richtstück 41 besitzt eine axiale Bohrung 47 mit halbkugelförmigem Bohrgrund 49 von der Gelenkkopfseite her und eine konische Langlochbohrung 51 durch die Anlenkfläche 43. Die Längsrichtung der Langlochbohrung 51 ist senkrecht zur Achsrichtung der Anlenkfläche 43 gerichtet, die konische Ausbildung des Langloches 51 besteht lediglich an dessen Enden.

Schaftteil 11, Drehstück 33 und Richtstück 41 sind mit den einander entsprechenden Passformen, Anlenkfläche 25 am Schaftteil und Kegelstumpf mit Kegelmantel 34 am Drehstück 33 einerseits, sowie zylindrische Ausnehmungsfläche 35 am Drehstück und Anlenkfläche 43 am Richtstück 41 andererseits, zusammenfügbar und mit einer Schraube 53 zusammenschraubbar. Die Schraube 53 besitzt einen Schaft 55 mit einem Gewindeabschnitt 57 und einem glatten Schaftteil 59, sowie einem kugelförmigen Schraubenkopf 61. Der Kugelradius des Schraubenkopfes entspricht dem Kugelradius des kugelförmigen Bohrgrundes 49 des Richtstückes 41. Der Schraubenkopf kann auch eine von der Kugelform abweichende Form aufweisen, wie dies in Fig. 1a gezeigt ist. Das Richtstück 41 ist aus einem Walzenstück 52 herausgearbeitet. Der Radius des Walzenstücks ist der Radius der Anlenkfläche 43.

Soll nun der Schaftteil 11 und das Richtstück 41 zusammengesetzt werden, so wird zuerst das Drehstück 33 mit der zylindrischen Ausnehmung nach aussen in die kegelstumpfförmig vertiefte Anlenkfläche 25 im Schaftteil 11 und danach das Richtstück 41 mit seiner zylindrischen Anlenkfläche 43 in die zylindrische Oberfläche 35 der Ausnehmung eingesetzt. Sodann wird die Schraube 53 mit dem Schaft 55 voran in die Bohrung 47 eingeführt und der Schraubenschaft 55 durch die Langlochbohrung 51 im Richtstück sowie die Bohrung 39 im Drehstück 33 gestossen und in das Gewinde in der Bohrung 31 im Schaftteil 11 eingeschraubt. Ist die Schraube fest angezogen decken sich die Achsen 37 und 37', und der Mittelpunkt des kugelförmigen Schraubenkopfes 61 liegt auf dem Schnittpunkt der Achsen 37,37' mit der ersten Achse 27.

Ist die Schraube 53 nicht fest angezogen, so ist einerseits das Drehstück 33 um die erste Achse 27 im Schaftteil verdrehbar, wodurch die Richtung der zweiten Achse 37 gewählt werden kann, und andererseits das Richtstück 41 um die zweite Achse 37 im Drehstück 33 verschwenkbar. Durch Überlagerung dieser beiden Dreh- und Schwenkbewegungen ist die Richtachse 46 in alle Richtungen in einem wählbaren Winkel zur vorgewählten Halsachse, welche in diesem Ausführungsbeispiel der ersten Achse 27 entspricht, stellbar. Der Winkel zwischen erster Achse 27 und Richtachse 46 ist zwischen 0 und etwa 30 Grad wählbar. Der Winkel bezüglich der Rotation des Drehstücks um die erste Achse 27 ist zwischen 0 und 360 Grad wählbar.

Damit der Chirurg möglichst handliche Teile in der Hand hat, sind zweckmässigerweise kleiner Teile vor deren Implantation zusammengestellt und miteinander verbunden, so kann z.B. das Drehstück 33 im Schaftteil 11 eingelassen und darin fixiert oder gesichert sein. Die Schraube 53 kann im Richtstück 41 eingesetzt und darin gesichert sein. Es kann auch vorbereitend die Schraube 53, das Richtstück 41 und das Drehstück 33 zusammengesetzt und miteinander provisorisch verbunden werden. Weitere Möglichkeiten, die Anzahl der während der Operation zusammenzusetzenden Teile zu verkleinern, und die Kleinteile zusammenzufassen besteht darin, vorgängig das Richtstück 41 und das Drehstück 33 zusammenzusetzen, oder aber Richtstück 41, Drehstück 33 und Schaftteil 11, mit oder ohne Schraube 53 zusammenzusetzen. Dabei kann die Schraube 53 locker oder in einer provisorischen Stellung des Richtstücks 41 angezogen sein. Bei den anderen Ausführungsbeispielen sind analoge Kombinationen der Teile sinnvoll.

Auf das nach der optimalen Gelenkhalsrichtung gerichteten und in dieser Stellung mit der Schraube 53 fixierte Richtstück 41 ist der Gelenkprothesenkopf aufsetzbar. Der Gelenkprothesenkopf besteht im Ausführungsbeispiel gem. Figur. 1 aus einem Exzenterring 63 und einer Kopfkalotte 65 mit einem der ursprünglichen Artikulationsfläche des zu ersetzenden Schultergelenkes in etwa entsprechenden Radius. Der Exzenterring 63 besitzt zwei gleichgerichtete Kegelstumpfmantelflächen mit voneinander beabstandeten parallelen Achsen 46' und 67 (siehe auch Figur 3). Dieser Exzenterring 63 weist eine innere, zentrisch um die Achse 46' angeordnete Konusfläche 69, welche auf die Konusfläche 45 am Richtstück 41 abgestimmt ist, und eine äussere Konusfläche 71 mit der Exzenterachse 67 als Rotationszentrum auf. Die äussere Konusfläche 71 des Exzenterringes 63 ist auf die Konusfläche 73 in der Kopfkalotte 65 abgestimmt.

Die Kopfkalotte 65 ist vorteilhaft ein dem osteotomierten Gelenkkugelabschnitt ähnlicher Kugelabschnitt. Die Artikulationsoberfläche 75 der Kalotte 65 liegt bei einer idealisierten Kalotte auf einem Radius um ihren Kugelmittelpunkt 77. Die Kalotte 65 besitzt eine der Kugeloberfläche 75 gegenüberliegende ebene Unterseite 79. Die Ebene der Unterseite 79 schneidet geometrisch die theoretische Kugeloberfläche in einem Kreis. Durch den Mittelpunkt dieses Kreises und den Kugelmittelpunkt 77 ist eine Kalottenachse 81 bestimmt. Exzentrisch zur Kalottenachse 81 ist in der Unterseite 79 eine konische Vertiefung 83 vorgesehen, deren axial um die Exzenterachse 67' ausgebildete, konische Wandung 73 und deren Tiefe auf die Masse der äusseren Konusfläche 71 des Exzenterringes 63 abgestimmt sind. Die Kugeloberfläche 75 der Kalotte 65 ist dazu vorgesehen, mit der natürlichen Gelenkfläche des Glenoids oder mit einem implantierten Glenoidteil 85 zusammenzuarbeiten.

Der Gelenkkopf wird auf das Richtstück 41 aufgesetzt, indem die innere Konusfläche 69 des Exzenterring 63 auf die Konusfläche 45 des Verbindungsteils 44 gepresst und die Kalotte mit der Vertiefung 83 auf die äussere Konusfläche 71 des Exzenterringes aufgedrückt wird. Dabei fallen die Achsen 46 und 46' zusammen, wie auch die Exzenterachsen 67 und 67'. Durch Verdrehen des Exzenterringes um die Richtachse 46 und der Kalotte um die Exzenterachse 67 ist Richtung und Abstand zwischen den Achsen 46,46' und 67/67' mit Richtung und Abstand zwischen den Achsen 67,67' und 81 als Vektoren addierbar. Unter der Voraussetzung, dass die Achsabstände gleich gross sind, kann die Kalottenachse 81 mit einem wählbaren Abstand zur Längsachse 46 des Richtstückes 41 zwischen 0 und der Summe dieser Achsabstände angeordnet werden. Die Kalottenachse kann zudem in eine beliebige Drehstellung zwischen 0 und 360 Grad um die Richtachse 46 gebracht werden, ohne das Richtstück 41 zu drehen. Dadurch ist die Kalottenachse 81 nach der Fixierung der Richtung der Richtachse 46 exakt durch den ursprünglichen Gelenkkugelmittelpunkt richtbar.

Ist die Osteotomie derart ausgeführt, dass der Knochenschnitt entlang dem Rand der Gelenkkugel geführt ist, kann die Unterseite 79 der Kalotte in Bezug auf die Knochenschnittebene parallel ausgerichtet werden, indem das Richtstück 41 senkrecht zur Knochenschnittebene gerichtet wird. Die Lage der Kalottenachse 81 kann danach anhand der Umrisslinie des Knochenschnittes ermittelt werden. Die Position des Kugelmittelpunktes muss in der Folge noch in bekannter Weise durch ein Set von bezüglich der Kalottenhöhe verschiedenen Kopfkalotten 65 anhand der Muskelspannung eingestellt werden.

Figur 1a zeigt zwei Varianten 53a und 53b der Schraube 53 gemäss Figur 1. Diese weisen ebenfalls einen Schaftteil 57 mit Gewinde und einen glatten Schaftteil 59 auf, jedoch ist der Schraubenkopf von zylindrischer 61a bzw. von kegelstupfförmiger oder prismatischer 61b Gestalt. Dank dieser nicht kugelförmigen Gestalt des Schraubenkopfes 61a,b ergibt sich eine ringförmige, nahezu lineare Anpressfläche zwischen dem Schraubenkopf 61a,b und der kugeligen Ausnehmung 49 im Richtstück 41. Die Anpresskante 60a,b des Schraubenkopfes 61a,b kann dabei durch Fasenabtrag gerundet oder scharfkantig sein. Mit dieser Anpresskante 60a,b kann das Richtstück gegen das Drehstück 33 gehalten werden, ohne dass dadurch die freie Verschwenkbarkeit um das Kugelzentrum 37' beeinträchtigt wird. Die Kante 60a,b liegt in jeder Schwenkposition des Richtstückes 41 kreisförmig an der kugeligen Ausnehmung 49 an. Beim Festziehen der Schraube 53a,b jedoch wird diese Anpresskante 60a,b in die kugelige Ausnehmung 49 eingepresst, so dass die Fixierung des Richtstückes verlässlicher erfolgt als bei flächiger Auflage eines kugelförmigen Schraubenkopfes 61.

Umgekehrt ist es auch mit entsprechend ähnlicher Wirkung möglich, die Ausnehmung 49 mit einer zylindrischen oder kegelmantelförmigen Innenfläche 49a auszuformen, welche eine ringförmige Anpresskante 50 in einer Stirnfläche der Ausnehmung 49 des Richtstücks aufweist. Ein solches Richtstück 41a ist in Figur 1b dargestellt. Diese Anpresskante 50 wirkt mit einem kugelförmigen Schraubenkopf 61 zusammen. Die Anpresskanten 50 bzw. 60 können auch doppelt, dreifach oder vielfach ausgeführt sein. Sie müssen dazu lediglich auf einer theoretischen Kugeloberfläche um den Kugelmittelpunkt 37' liegen. Vorteilhaft liegen sie in einer zur Drehachse der Schraube senkrechten Ebene. Bei einer Vielzahl von Anpresskanten 50 oder 60 am Richtstück 41a oder an der Schraube 53a,b kann sowohl die Ausnehmung 49a als auch der Schraubenkopf 61a,b von der reinen Kugelform abweichen und Anpresskanten 50, 60 aufweisen.

In Figur 2 ist ein Langlochring 87 dargestellt. Der Langlochring 87 kann alternativ zum Exzenterring 63 verwendet werden. Dabei ist es bei der Verwendung eines Langlochringes 87 nicht notwendig, die konische Vertiefung 83 in der Kalottenunterseite 79 exzentrisch anzuordnen. Vielmehr ist diese vorteilhaft zentrisch angeordnet. Der Langlochring 87 besitzt analog zum Exzenterring 63 eine der konischen Vertiefung 83 in der Unterseite 79 der Kalotte 65 entsprechende äussere Konusfläche 71. Wenn die konische Vertiefung 83 zweckmässigerweise zentrisch in der Kalottenunterseite 79 angeordnet ist, kann die Konusfläche auch von einer strengen Kegelstumpfmantelfläche abweichend, z.B. als Pyramidenstumpf, geformt sein. Der Langlochring besitzt ein Langloch 89 parallel zur Exzenterachse 67", dessen Längsausdehnung sich quer zur Exzenterachse 67" erstreckt. Das Langloch 87 besitzt zwei halbzylinderförmige oder zwei halbkegelstumpfförmige Wandungen mit zwei ebenen Verbindungsflächen. Das Zentrum der einen halbbogenförmigen Wandung liegt auf der Exzenterachse 67", das Zentrum der anderen in einem gewählten Abstand dazu.

Das Langloch kann konisch sein, wenn der in das Langloch einzuschiebende Richtstück 41 einen Konus 45 aufweist. Das Richtstück 41 kann aber auch einen zylindrischen Verbindungsteil 44 aufweisen. Dies bedingt jedoch Feststellmittel im Exzenterring 63 bzw. im Langlochring 87. Im Langlochring 87 sind Feststellmittel, insbesondere zwei Madenschrauben 91 angeordnet. Die eine ist senkrecht zur Längsrichtung des Langloches auf das Zentrum der einen Halbbogenwandung, die andere auf dasjenige der anderen gerichtet. Dadurch lässt sich ein in das Langloch 89 eingeführter Verbindungsteil 44 des Richtstückes 41 in einer wählbaren Rotationslage und Verschiebelage im Langloch fixieren.

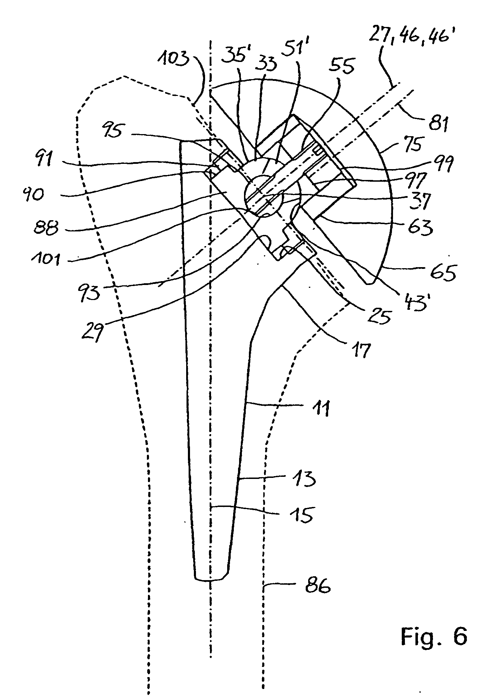

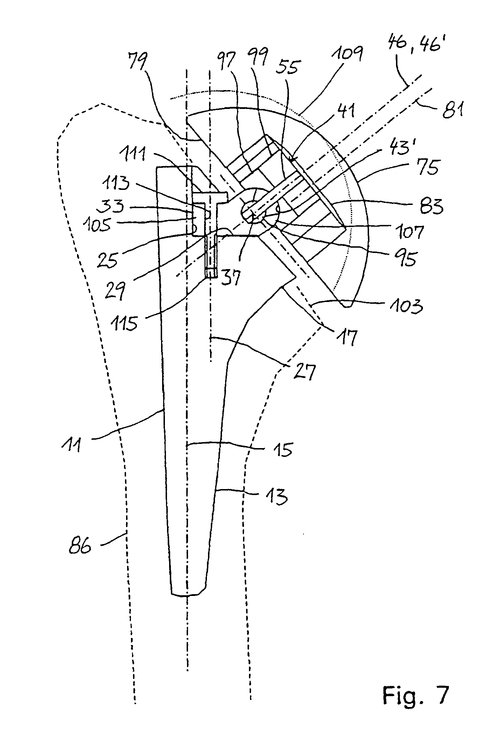

Die Figuren 3 bis 5 sind oben im Zusammenhang mit dem Ausführungsbeispiel gemäss Figur 1 beschrieben. In den Figuren 6 und 7 sind zwei weitere Ausführungsbeispiele dargestellt. Die entsprechenden Teile sind dabei mit den gleichen Bezugsziffem bezeichnet, auch wenn die Teile z.T. wesentlich abweichende Formen aufweisen.

Im schematischen Schnitt gemäss Figur 6 ist ein zweites Ausführungsbeispiel dargestellt, bei welchem zwar wie im ersten Beispiel die erste Achse 27 der mittleren Halsachse entspricht und sich die erste Achse 27 und die zweite Achse 37 in einem Punkt schneiden, welcher nach der Implantation des Schaftes in den Humerusknochen 86 möglichst in der Knochenschnittebene 103 der abgetrennten Gelenkkugel liegt. Bei diesem Ausführungsbeispiel weist das Drehstück 33 aber eine konvexe Zylinderfläche 35 auf. Auch ist das Drehstück 33 unabhängig von der Fixierung der Kippbewegung des Richtstückes 41 bezüglich Drehstück 33 am Schaftteil 11 fixierbar. Dazu ist folgende Konstruktion vorgesehen:

Im Schaftteil 11, insbesondere im Schaftkopf 17 ist eine zylindrische Vertiefung 25 vorgesehen, deren Zylinderachse 27, das ist die erste Achse, etwa nach der natürlichen Halsachse ausgerichtet ist. Diese Vertiefung 25 ist mit einem Gewinde versehen. In dieser Vertiefung 25 ist das Drehstück 33 um die erste Achse 27 drehbar angeordnet. Das Drehstück 33 weist eine Basis 88 mit einem kreisrunden Rand 90 auf, welcher Rand 90 seitlich über eine halbzylindrische Aufwölbung hinaussteht. Diese Aufwölbung ist die Anlenkfläche 35' für das Richtstück 41. Dieser Rand 90 arbeitet mit einer in das Gewinde in der Vertiefung 25 eingeschraubten Ringschraube 91 derart zusammen, dass durch Anziehen der Ringschraube 91 der Rand 90 gegen den Boden 29 der Vertiefung 25 gepresst wird. Dadurch wird das Drehstück 33 in der Vertiefung 25 fixiert. Die Achse 37 der halbzylindrischen Aufwölbung 35, d.h. die zweite Achse, verläuft senkrecht durch die erste Achse 27. Konzentrisch mit der Aufwölbungsfläche 35 ist im Drehstück 33 eine Bohrung 93 vorgesehen. In der Bohrung 93 steckt ein zylinderförmiges Körper 95, welcher die Bohrung im Durchmesser ausfüllt. Dieser Körper bzw. dieses Kopfteil 95 ist um die Achse 37 in der Bohrung drehbar. Mit dem Kopfteil 95 ist ein Schraubenschaft 55 verbunden. Der Schraubenschaft steht senkrecht zur zweiten Achse 37. In der Figur 6 ist der Schraubenschaft 55 sogar nach der mittleren Halsachse ausgerichtet Der Schraubenschaft 55 durchstösst die Wandung zwischen Bohrung 93 und Anlenkfläche 35'. Dazu ist in dieser Wandung am Drehstück 33 eine konische Langlochbohrung 51' vorgesehen. Die Längsrichtung der Langlochbohrung 51' ist senkrecht zur Achsrichtung der Anlenkfläche 35' gerichtet, die konische Ausbildung des Langloches 51' besteht lediglich an dessen Enden. Das Langloch erlaubt ein Verdrehen von Kopfteil 95 zusammen mit dem Schraubenschaft 55 um die zweite Achse 37.

Auf den Schraubenschaft 55 sind eine Anlenkscheibe 97 und eine die Aufgabe des Verbindungsteils 44 übernehmende Druckscheibe 99 aufgesetzt. Die Anlenkscheibe 97 und die Druckscheibe 99 bilden zusammen das sowohl um die erste wie die zweite Achse verschwenkbare Richtstück 41. Die Anlenkscheibe 97 hat einen kreisrunden Umriss mit der Achse 46 und besitzt eine der Anlenkfläche 35' am Drehstück entsprechende konkave Zylinderfläche 43' auf der einen Seite, und auf der gegenüberliegenden Fläche eine Druckfläche, auf welche die Druckscheibe 99 flächig aufpressen kann. Axial ist in der Anlenkscheibe 97 eine Bohrung ausgebildet, durch welche der Schraubenschaft 55 hindurchpasst. Die Druckscheibe 99 hat einen kreisrunden Umriss mit der Achse 46. Axial ist eine Gewindebohrung ausgebildet, mit welcher die Druckscheibe 99 auf den Schraubenschaft 55 aufschraubbar ist. Die Druckscheibe 99 kann alternativ auch mit dem Schraubenschaft 55 fest verbunden sein, was jedoch eine Gewindeverbindung zwischen Schraubenschaft und Kopfteil 95 bedingt.

Diese Gewindeverbindung zwischen Kopfteil 95 und Schraubenschaft 55 ist jedoch auch sonst vorteilhaft, weil sie ein erstes Fixieren des Kopfteiles 95 im Drehstück 33 erlaubt, bevor das Richtstück 41 auf dem Schraubenschaft 55 angeordnet ist. Durch Festziehen des Schraubenschaftes im Kopfteil 95 kann dieses einfach mit dem Drehstück verklemmt werden. Ist jedoch schaftteil- bzw. kopfteilseitig am Schraubenschaft 55 eine Spitze 101 ausgebildet, bohrt sich diese beim Anziehen der Schraube in das Drehstück 33, so dass die Drehbarkeit des Richtstückes um die zweite Achse 37 sicher verhindert ist.

Alternativ kann das Drehstück 33 derart ausgebildet sein, dass die Bohrung 93 zum Schaftteil 11 hin offen ist und das Kopfteil 95 nur auf der Seite des Richtstücks 41 umfasst, zum Boden 29 der Vertiefung 25 im Schaftteil 11 hin jedoch frei lässt. Das Kopfteil 95 sitzt dann beispielsweise in einem parallel zur zweiten Achse 37 ausgebildeten Schlitz mit der Breite und Höhe des Kopfstückes 95 und einem halbzylindrischen Schlitzgrund. Dies erlaubt das Einpressen der Schraubenschaftspitze in den Boden 29. Der Boden kann dazu in diesem Bereich eine konkave Kugeloberfläche mit dem Zentrum im Schnittpunkt der ersten und der zweiten Achse aufweisen, so dass die Spitze 101 senkrecht zur Oberfläche des Bodens 29 in diese hineinsticht. Dies behindert auch die unabsichtliche Verdrehung des Drehstücks um die erste Achse.

Durch Aufschrauben des Verbindungsteils 99 auf den Schraubenschaft 55 oder durch Festschrauben des am Verbindungsteil befestigten Schraubenschaftes im Kopfteil 95 werden jedenfalls Kopfteil 95 und Anlenkscheibe 97 zusammengezogen. Dadurch kann das Richtstück 41 am Drehstück 33 in jeder Schwenklage fixiert werden.

Die Druckscheibe 99 weist eine äussere Konusfläche 45 auf, auf welcher ein Exzenterring 63 aufgesetzt ist. Auf dem Exzenterring 63 sitzt die Kopfkalotte 65. Durch die Exzentrizität von Exzenterachsen 46', 67 und der Achsen 67' und 81 der konischen Vertiefung 83 an der Kalotte 65 und der Kopfkalotte 65 kann die Kalottenachse 81 bezüglich der Richtachse 46 in einer wählbaren Richtung und einem wählbaren Abstand angeordnet werden (Exzenterachsen 67,67' sind nicht eingezeichnet). Dabei ist die Richtachse 46 bezüglich der ersten Achse 27 (oder der Schaftachse 15) in einer wählbaren Richtung und um einen wählbaren Winkel verschwenkbar. Alternativ kann auch der Exzenterring entfallen und die Kopfkalotte direkt auf die Konusoberfläche des Richtstücks aufgesetzt werden.

Dank diese Einstellbarkeit der Achsen lässt sich in einem ersten Schritt die Richtachse 46 senkrecht zur Knochenschnittfläche 103 einstellen und das Richtstück und das Drehstück in dieser Lage fixiert werden. In einem zweiten Schritt kann dann die Lage der Kalottenachse 81 bezüglich der Richtachse 46 auf den Knochenschnittumriss abgestimmt werden und die Kalotte 65 auf das Richtstück 41 festgeschlagen werden.

Das dritte Ausführungsbeispiel gemäss Figur 7 macht schliesslich deutlich, dass die erste und die zweite Achse sich nicht unbedingt in einem gemeinsamen Drehpunkt in der oder nahe der Knochenschnittebene 103 schneiden müssen. Die Achsen 27 und 37 können rechtwinklig oder schiefwinklig zueinander verlaufen und voneinander beabstandet angeordnet sein. Im dritten Ausführungsbeispiel ist die erste Achse 27 nicht gemäss der natürlichen Halsachse gerichtet, sondern parallel zur Schaftachse 15. Dadurch ist die Ausnehmung 25 im Schaftteil 11, in welcher das Drehstück 33 gelagert und fixierbar ist, orthogonal zur Schaftachse 15 gerichtet. Das Drehstück 33 weist einen schaftseitigen Anlenkteil 105 und einen richtstückseitigen Anlenkteil 107 auf. Der richtstückseitige Anlenkteil 107 ist analog zur zylindrischen Auswölbung 35' des zweiten Ausführungsbeispiels ausgebildet und mit einer konvexen Anlenkfläche 35' versehen, könnte aber auch analog zum ersten Ausführungsbeispiel eine konkave Anlenkfläche 35 aufweisen. Es ist auch hier in einer mit der zylindrischen Anlenkfläche 35' konzentrischen zylindrischen Bohrung 93 ein zylinderförmiges Kopfteil 95 angeordnet. Durch eine Langlochbohrung 51' in der Wandung mit der Anlenkfläche 35' hindurch ist der Schraubenschaft 55 in das Kopfteil 95 eingeschraubt. Der Schraubenschaft 55 besitzt dadurch eine Schwenkfreiheit entsprechend der Länge der Langlochbohrung 51'. Auf dem Schraubenschaft ist wie im zweiten Ausführungsbeispiel eine Anlenkscheibe 97 und eine Druckscheibe 99 angeordnet. Die Druckscheibe 99 bildet mit der Anlenkscheibe 97 zusammen das Richtstück 41.

Alternativ kann das Richtstück durch die Druckscheibe 99 allein gebildet sein. In diesem Fall ist die Anlenkscheibe 97 zweckmässigerweise mit einem kleineren Durchmesser ausgebildet als die Druckscheibe 99. Auf dem Richtstück 41 sitzen zwei Exzenterringe 63,64. Mit den beiden Exzenterringen kann die Lage der Kalottenachse 81 eingestellt werden. Daher weist die Kalotte 65 eine mit dem zweiten Exzenterring 64 zusammenwirkende Ausnehmung 73 in ihrer Unterseite 79 auf, welche zentrisch auf der Kalottenachse 81 liegt. Durch Verdrehen der Exzenterringe 63,64 kann die äussere Konusfläche 71' des äusseren Exzenterringes 64 relativ zur Umrisslinie des Knochenschnittes derart angeordnet werden, dass die Kalotte 65 nach dem Aufsetzen auf den Exzenterring 64 in Bezug auf die Lage der ursprünglichen Artikulationsflächenachse optimal sitzt.

Die Rotationslage wird durch Anziehen der Schraube 111 fixiert. Die Schraube 111 steht axial zur ersten Achse 27 und bildet die Schwenkachse für das Drehstück 33 gegenüber dem Schaftteil 11. Das Drehstück besitzt dazu in seinem schaftseitigen Anlenkteil 105 eine axiale Bohrung 113, durch welche die Schraube 111 in eine Gewindebohrung 115 im Schaftteil 11 hineinreicht. Die Fixation geschieht durch den Druck, den mit dem Schraubenkopf der Schraube 111 auf das schaftseitige Anlenkteil 105 ausgeübt wird, bzw. durch das Zusammenpressen der Berührungsflächen zwischen Drehstück 33 und Schaftteil 11.

Die Anlenkscheibe 97 ist mit einer ebenflächigen Anlenkfläche 43" ausgebildet. Diese ermöglicht ein Verpressen von Anlenkscheibe und Drehstück unter Materialverformung, wodurch ein sehr guten Halt erzielt werden kann.

Dank der Einstellbarkeit der Lage der Kalottenachse 81 bezüglich der Richtachse 46 ist die Kalotte 65, wie durch die punktierte Linie 109 zeigt, bezüglich des Humerusknochens 86 bzw. dem Umriss von dessen Schnittfläche 103 verschiebbar. Dies erlaubt eine bezüglich der angestrebten Lage der Kopfkalotte 65 ungefähre, nicht präzise Implantation des Schaftteils 11 in den Knochen 86 einerseits, aber auch eine Verschiebung der Richtachse 46 bei der Einstellung des Winkels der Richtachse 46 zur Schaftachse 15 oder der Rotationslage der Richtachse 46 bezüglich dem Humerusknochen 86.

Durch die Einstellung der Rotationslage des Richtstückes 41 verschiebt sich im dritten Ausführungsbeispiel denn auch die Lage der Durchtrittstelle der Richtachse 46 durch die Oberfläche des Knochenschnittes 103. Eine Rotation nach anterior bewirkt eine Verschiebung der Durchtrittstelle nach anterior. Hingegen bewirkt eine Verschwenkung der Richtachse 46 nach cranial, d.h. in Figur 7 im Gegenuhrzeigersinn, ein Verschiebung der Durchtrittstelle der Richtachse durch die Knochenschnittebene 103 nach caudal, d.h. in Figur 7 nach unten rechts.

Dankdem ein bezüglich dem Schaftteil 11 um eine erste Achse 27 verdrehbares Drehstück 33 am Schaftteil angelenkt ist, und an diesem Drehstück 33 ein Richtstück 41 um eine zweite Achse 37 verschwenkbar angeordnet ist, welche zweite Achse 37 quer zur ersten Achse 27 steht, ist ein Ausrichten des Richtstückes bezüglich der Knochenschnittebene 103 in alle Richtungen möglich. Dadurch entstehende Verschiebungen der Durchtrittstelle der Richtachse 46 durch die Knochenschnittebene 103 können im gleichen Zug korrigiert werden, mit welchem die Einstellung der Kalottenachse 81 auf die wünschbare, individuelle Stellung bezüglich Knochenschnitt bzw. ursprünglicher Kalottenlage vorgenommen wird. Dadurch können alle notwendigen Parameter eingestellt werden, so dass die Kopfkalotte 65 schliesslich an die optimale, der ursprünglichen Stelle entsprechende Stelle gesetzt werden kann.

Die Einstellung der Muskelspannung geschieht wie herkömmlich durch die Wahl einer Kalotte aus einem Set von Kalotten mit unterschiedlichen Kalottenhöhen. Es ist aber auch möglich, die definitive Kalottenhöhe dadurch einzustellen, dass zwischen Anlenkscheibe 97 und Druckscheibe 99 eine Distanzscheibe 117 eingefügt wird. Durch die Verwendung solcher Distanzscheiben oder Druckscheiben unterschiedlicher Höhe, welche günstiger in der Herstellung sind als Kopfkalotten, erübrigt sich die Herstellung und Bereitstellung eines ganzen Sets von Kopfkalotten mit unterschiedlichen Kalottenhöhen. Weiter ist durch die zentrische Anordnung der Vertiefung 73 in der Unterseite 79 der Kalotte 65 auch die Möglichkeit gegeben, die Kalotte 65 mit einem Gewinde auf den äusseren Exzenterring 64 oder den Langlochring 87 aufzuschrauben. Dies erlaubt eine stufenlose Einstellung der Kalottenhöhe, bedingt aber eine Feststelleinrichtung, z.B. in Form einer zuerst auf das Gewinde aufgeschraubten Gegenmutter, welche gegen die Unterseite 79 der Kalotte 65 angezogen werden kann.

Zusammenfassend gesagt, weist eine Endoprothese für ein Schultergelenk ein an einem Schaftteil 11 um eine erste Achse 27 drehbar angelenktes Drehstück 33 und ein am Drehstück 33 um eine zweite Achse angelenktes axiales Richtstück 41 auf. Die beiden Schwenkachsen 27,37 stehen quer zueinander und die zweite Achse 37 steht quer zur Achse 46 des Richtstückes 41. Auf dem Richtstück 41 ist die Kopfkalotte 65 entweder direkt angeordnet, oder zwischen Kopfkalotte 65 und Richtstück 41 ist ein Exzenterring 63 oder ein Langlochring 87 vorgesehen, welcher die Kopfkalotte 65 trägt. Auch zwei oder mehr Exzenterringe 63,64 sind möglich. Die Kopfkalotte 65 kann jeweils eine zentrische oder exzentrische Konusfläche 73 aufweisen. Die Richtung der ersten Achse 27 kann relativ frei gewählt werden, d.h. z.B. parallel zur Schaftachse 15, etwa parallel zur mittleren Ausrichtung der Richtachse 46, etwa parallel zur Ebene des Knochenschnittes, insbesondere auch etwa senkrecht zur Richtachse 46 und zur zweiten Achse 37. Sie schneidet vorteilhaft die Richtachse 46 z.B. in deren mittleren Ausrichtung.

## Patentansprüche

1. Endoprothese für ein Schultergelenk,
mit einem Schaftteil (11), welches einen axialen Schaftstiel (13) zum Implantieren in den Humerus (86) und einen Schaftkopf (17) aufweist,
mit einer Anlenkfläche (25,29,35,35') im Bereich des Schaftkopfs (17),
und einem am Schaftkopf (17) angelenkten, entlang einer Richtachse (46) sich erstreckendes Richtstück (41), welches bezüglich des Schaftteils (11) in alle Richtungen verschwenkbar ist und in einer wählbaren Schwenkstellung relativ zum Schaftteil (11) fixierbar ist,
sowie einer über das Richtstück (41) mit dem Schaftteil (11) verbindbaren Kopfkalotte (65),
**dadurch gekennzeichnet,**
**dass** zwischen Schaftkopf (17) und Richtstück (41) ein Drehstück (33) angeordnet ist,
welches gegenüber dem Schaftteil (11) um eine erste Achse (27) verdrehbar ist, dass das Richtstück (41) bezüglich des Drehstücks (33) um eine zweite Achse (37) drehbar gelagert ist,
**dass** die zweite Achse (37) quer zur ersten Achse (27) und quer zur Richtachse (46) verläuft,
und **dass** Anlenk- oder Berührungsflächen (25,29,35,35',43,43',43") zwischen Schaftteil (11) und Drehstück (33), und zwischen Drehstück (33) und Richtstück (41) jeweils lediglich eine Relativbewegung zwischen Schaftteil (11) und Drehstück (33), beziehungsweise zwischen Drehstück (33) und Richtstück (41) um die jeweilige gemeinsame erste (27) bzw. zweite (37) Achse als Rotationszentrum zulassen.

2. Endoprothese nach Anspruch 1, **gekennzeichnet durch** wenigstens einen vom Richtstück (41) unabhängigen Exzenterring (63,64) oder Langlochring (87) zwischen Richtstück (41) und Kopfkalotte (65).

3. Endoprothese nach Anspruch 1 oder 2, **dadurch gekennzeichnet, dass** das Richtstück (41) eine äussere Konus- oder Zylinderoberfläche (45) aufweist, deren Rotationszentrum die Richtachse (46) ist, auf welche Konus- oder Zylinderoberfläche (45) die Kopfkalotte (65), der Exzenterring (63,64) oder der Langlochring (87) aufsteckbar ist.

4. Endoprothese nach einem der Ansprüche 1 bis 3, **dadurch gekennzeichnet, dass** die Richtung der ersten Achse (27) eine Richtungsabweichung von der Richtung der Halsachse um weniger als 60 Grad, vorzugsweise weniger als 50 Grad aufweist.

5. Endoprothese nach einem der Ansprüche 1 bis 4, **dadurch gekennzeichnet, dass** die Richtung der ersten Achse (27) praktisch parallel zu einer Ebene durch Schaftachse (15) und Halsachse liegt.

6. Endoprothese nach einem der Ansprüche 1 bis 5, **dadurch gekennzeichnet, dass** das bezüglich des Schaftteils (11) um die erste Achse (27) verdrehbare Drehstück (33) eine gegen das Richtstück (41) gerichtete konkave axiale Rotationskörperoberfläche (35) mit der zweiten Achse (37) als Rotationszentrum aufweist.

7. Endoprothese nach einem der Ansprüche 1 bis 5, **dadurch gekennzeichnet, dass** das bezüglich dem Schaftteil um die erste Achse verdrehbare Drehstück (33) eine gegen das Richtstück (41) gerichtete konvexe Rotationskörperoberfläche (35') mit der zweiten Achse (37) als Rotationszentrum aufweist

8. Endoprothese nach einem der Ansprüche 1 bis 7, **dadurch gekennzeichnet, dass** die erste Achse (27) die zweite Achse (37) in einem Zentralpunkt schneidet.

9. Endoprothese nach einem der Ansprüche 1 bis 7, **dadurch gekennzeichnet, dass** die beiden Achsen (27,37) voneinander beabstandet sind.

10. Endoprothese nach einem der Ansprüche 1 bis 9, **dadurch gekennzeichnet, dass** die erste Achse (27) quer zur Halsachse steht.

11. Endoprothese nach einem der Ansprüche 1 bis 9, **dadurch gekennzeichnet, dass** die erste Achse (27) parallel zur Halsachse steht.

12. Endoprothese nach einem der Ansprüche 1 bis 11, **dadurch gekennzeichnet, dass** die erste Achse (27) die Halsachse schneidet oder mit ihr zusammenfällt.

13. Endoprothese nach einem der Ansprüche 1 bis 12, **dadurch gekennzeichnet, dass** die Drehbewegungen um die erste (27) und die zweite Achse (37) einzeln fixierbar sind.

14. Endoprothese nach einem der Ansprüche 1 bis 13, **dadurch gekennzeichnet, dass** die Drehbewegungen um die erste (27) und die zweite Achse (37) gemeinsam fixierbar sind.

15. Endoprothese nach Anspruch 2, allenfalls in Verbindung mit einem der Ansprüche 3 bis 14, bei welcher ein einzelner Exzenterring (63) Verwendung findet, **dadurch gekennzeichnet, dass** die Kopfkalotte (65) eine bezüglich der Kalottenachse (81) exzentrisch angeordnete innere Konusoberfläche (73) aufweist, welche auf die äussere Konusoberfläche (71) des Exzenterringes (63) aufsteckbar ist.

16. Endoprothese nach Anspruch 15, **gekennzeichnet durch** einen Manipulierkopf, welcher eine bezüglich der Kalottenachse (81) exzentrisch angeordnete innere Konusoberfläche (73) aufweist, welche auf die äussere Konusoberfläche (71) des Exzenterringes (63) aufsteckbar ist und deren Exzentrizität der Exzentrizität der inneren Konusoberfläche (73) der Kopfkalotte entspricht, und welcher derart ringförmig ausgestaltet ist, dass der Exzenterring (63) **durch** die Ringöffnung des Manipulierkopfes hindurch sichtbar und verstellbar ist.

17. Endoprothese nach Anspruch 2, allenfalls in Verbindung mit einem der Ansprüche 3 bis 15, **gekennzeichnet durch** einen Langlochring (87), welcher ein Langloch (89) aufweist, mit welchem er auf einen Verbindungsteil (44) des Richtstücks (41) aufsetzbar ist, und mit einer Feststelleinrichtung (91), mit welcher der Langlochring (87) in einer beliebigen Verschiebe- und Drehstellung bezüglich dem Richtstück (41) an diesem fixierbar ist.

18. Endoprothese nach Anspruch 17, **dadurch gekennzeichnet, dass** die mit dem Langlochring (87) zusammenwirkende Vertiefung (73) in der Kopfkalotte (65) konzentrisch mit der Kalottenachse (81) angeordnet ist.

19. Endoprothese nach einem der Ansprüche 1 bis 16, **dadurch gekennzeichnet, dass** im Schaftteil (11) eine Ausnehmung (25) vorliegt mit einer Seitenwandung (25), welche einer Rotationsfläche um die erste Achse (27) entspricht, darin ein Drehstück (33) mit einem der Seitenwandung (25) entsprechenden Umriss um die erste Achse (27) drehbar gelagert ist, welches Drehstück (33) eine Anlenkfläche (35,35') für das Richtstück (41) in Form einer Rotationsfläche um die zweite Achse (37) aufweist, das Richtstück (41) eine dieser Anlenkfläche entsprechende Gegenform (43,43') und eine axiale Konusoberfläche (45) zum Aufstecken einer inneren Konusoberfläche (69) der Kopfkalotte (65) oder eines Exzenterringes (63) aufweist.

20. Endoprothese nach Anspruch 19, **dadurch gekennzeichnet, dass** im Schaftteil (11) eine Gewindebohrung (31) vorgesehen ist und das Richtstück (41) eine Innenbohrung (47) aufweist, in welcher der Kopf (61) einer Schraube (53) Platz findet, dass das Drehstück (33) und das Richtstück (41) eine Bohrung (39,51) aufweisen, und dass das Richtstück (31) mit der Schraube (53) durch die Bohrungen (39,51) hindurch in der Gewindebohrung (31) festschraubbar ist.

21. Endoprothese nach Anspruch 20, **dadurch gekennzeichnet, dass** die Bohrung (51) im Richtstück (41) für den Schraubenschaft (55) als Schlitz ausgebildet ist, welcher einen Bewegungsbereich des Schraubenschaftes (55) von ± 30° zulässt und dessen Breite dem Durchmesser des Schraubenschaftes (55), insbesondere eines mit der Bohrung (51) zusammenwirkenden gewindelosen Teils (59) desselben, angepasst ist.

22. Endoprothese nach einem der Ansprüche 1 bis 21, **dadurch gekennzeichnet, dass** der Radius der Anlenkfläche (43) des Richtstückes (41) so gross gewählt ist, dass das gesamte Richtstück (41) inklusive Verbindungsteil (44) innerhalb eines Zylinderstückes mit diesem Radius liegt.

23. Endoprothese nach Anspruch 7, allenfalls in Verbindung mit einem der Ansprüche 8 bis 22, **dadurch gekennzeichnet, dass** im Drehstück (33) eine mit der Anlenkfläche (35') um die zweite Achse (37) konzentrische Bohrung (93) vorgesehen, und dass in der Bohrung (93) ein zylinderförmiger Körper (95) angeordnet ist, welcher Körper (95) mit einem Schraubenschaft (55) verbunden ist, die Wandung zwischen Bohrung (93) und Anlenkfläche (35') einen quer zur zweiten Achse (37) verlaufenden Schlitz (51') aufweist, durch welchen der Schraubenschaft (55) hindurchsteht, und dass das Richtstück (41) eine Anlenkscheibe (97) und eine Druckscheibe (99) aufweist, wobei die Anlenkscheibe (97) eine Bohrung für den Schraubenschaft (55) und eine der Anlenkfläche (35') des Drehstücks (33) entsprechende Anlenkfläche (43'), sowie auf der dieser gegenüberliegenden Seite eine mit der Druckscheibe (99) zusammenwirkende Druckfläche aufweist, und dass die Druckscheibe (99) mit dem Schraubenschaft (55) in Verbindung steht, wobei mit einer relativen Verdrehung zwischen Schraubenschaft (55) und zylindrischem Körper (95) und/oder Druckscheibe (99) die Distanz zwischen zylindrischem Körper (95) und Druckscheibe (99) veränderbar ist

24. Endoprothese nach einem der Ansprüche 19 bis 23, **dadurch gekennzeichnet, dass** das Drehstück (33) mit einem auf oder in das Schaftteil (11) schraubbaren Ring (91) am Schaftteil (11) fixierbar ist.

## Claims

1. An endoprosthesis for a shoulder joint,
having a shaft part (11), which has an axial shaft stem (13) for implantation into the humerus (86), and a shaft head (17),
having an articulation face (25, 29, 35, 35') in the region of the shaft head (17), and a directional piece (41), which is pivotably connected at the shaft head (17) and extends along a directional axis (46) and is pivotable in all directions relative to the shaft part (11) and is fixable in a selectable pivoted position relative to the shaft part (11),
and a spherical cap (65) that can be connected to the shaft part (11) via the directional piece (41),
**characterized in that**
a rotating piece (33) is disposed in between the shaft head (17) and the directional piece (41),
which rotating piece is rotatable relative to the shaft part (11) about a first axis (27);
that the directional piece (41) is supported rotatably relative to the rotating piece (33) about a second axis (37);
that the second axis (37) extends transversely to the first axis (27) and
transversely the directional axis (46);
and that articulation or contact faces (25, 29, 35, 35', 43, 43', 43") between the shaft part (11) and the rotating piece (33), and between the rotating piece (33) and the directional piece (41) each permit only a relative motion between the shaft part (11) and the rotating piece (33), or between the rotating piece (33) and the directional piece (41), about the common first axis (27) or the common second axis (37), as applicable, as a center of rotation.

2. The endoprosthesis of claim 1, **characterized by** at least one eccentric ring (63, 64) or oblong slot ring (87), independent of the directional piece (41), between the directional piece (41) and the spherical cap (65).

3. The endoprosthesis of claim 1 or 2, **characterized in that** the directional piece (41) has an outer conical or cylindrical surface (45), whose center of rotation is the directional axis (46), onto which conical or cylindrical surface (45) the spherical cap (65), the eccentric ring (63, 64) or the oblong slot ring (87) can be slipped.

4. The endoprosthesis of one of claims 1 to 3, **characterized in that** the direction of the first axis (27) has a directional deviation from the direction of the neck axis of less than 60 degrees, preferably less than 50 degrees.

5. The endoprosthesis of one of claims 1 to 4, **characterized in that** the direction of the first axis (27) is practically parallel to a plane through the shaft axis (15) and neck axis.

6. The endoprosthesis of one of claims 1 to 5, **characterized in that** the rotating piece (03), which is rotatable relative to the shaft part (11) about the first axis (27), has a concave axial rotational body surface (35), facing the directional piece (41), with the second axis (37) as its center of rotation.

7. The endoprosthesis of one of claims 1 to 5, **characterized in that** the rotating piece (33), which is rotatable relative to the shaft part (11) about the first axis (27), has a convex axial rotational body surface (35'), facing the directional piece (41), with the second axis (37) as its center of rotation.

8. The endoprosthesis of one of claims 1 to 7, **characterized in that** the first axis (27) intersects the second axis (37) at a central point.

9. The endoprosthesis of one of claims 1 to 7, **characterized in that** the two axes (27, 37) are spaced apart from one another.

10. The endoprosthesis of one of claims 1 to 9, **characterized in that** the first axis (27) is transverse to the neck axis.

11. The endoprosthesis of one of claims 1 to 9, **characterized in that** the first axis (27) is parallel to the neck axis.

12. The endoprosthesis of one of claims 1 to 11, **characterized in that** the first axis (27) intersects or coincides with the neck axis.

13. The endoprosthesis of one of claims 1 to 12, **characterized in that** the rotary motions about the first axis (27) and the second axis (37) are individually fixable.

14. The endoprosthesis of one of claims 1 to 13, **characterized in that** the rotary motions about the first axis (27) and the second axis (37) are fixable in common.

15. The endoprosthesis of claim 2, if need be in combination with one of claims 3 to 14, in which a single eccentric ring (63) is used, **characterized in that** the spherical cap (65) has an inner conical surface (73), which is disposed eccentrically relative to the spherical cap axis (81) and which can be slipped onto the outer conical surface (71) of the eccentric ring (63).

16. The endoprosthesis of claim 15, **characterized by** a manipulation head, which has an inner conical surface (73), disposed eccentrically relative to the spherical cap axis (81), which can be slipped onto the outer conical surface (71) of the eccentric ring (63), and whose eccentricity is equivalent to the eccentricity of the inner conical surface (73) of the spherical cap, and which is shaped annularly in such a way that the eccentric ring (63) is visible and adjustable through the annular opening of the manipulation head.

17. The endoprosthesis of claim 2, if need be in combination with one of claims 3 to 15, **characterized by** an oblong slot ring (87), which has an oblong slot (89) with which it can be placed onto a connecting part (44) of the directional piece (41), and having a fixation device (91), with which the oblong slot ring (87) is fixable on the directional piece (41) in an arbitrary displacement and rotational position relative to the directional piece.

18. The endoprosthesis of claim 17, **characterized in that** the indentation (73) in the spherical cap (65), cooperating with the oblong slot ring (87), is disposed concentrically with the spherical cap axis (81).

19. The endoprosthesis of one of claims 1 to 16, **characterized in that** there is a recess (25) in the shaft part (11), with a side wall (25) that corresponds to a face of rotation about the first axis (27), in which a rotating piece (33) with an outline corresponding to the side wall (25) is supported rotatably about the first axis (27), which rotating piece (33) has an articulation face (35, 35') for the directional piece (41), in the form of a face of rotation about the second axis (37), and the directional piece (41) has a complimentary shape (43, 43') to this articulation face and an axial conical surface (45) for slipping on an inner conical surface (69) of the spherical cap (65) or of an eccentric ring (63).

20. The endoprosthesis of claim 19, **characterized in that** a threaded bore (31) is provided in the shaft part (11), and the directional piece (41) has an internal bore (47), in which there is space for the head (61) of a screw (53); that the rotating piece (33) and the directional piece (41) have a bore (39, 51); and that the directional piece (31) can be firmly screwed with the screw (53) in the threaded bore (31), through the bores (39, 51).

21. The endoprosthesis of claim 20, **characterized in that** the bore (51) in the directional piece (41) for the screw shaft (55) is embodied as a slot, which allows a range of motion of the screw shaft (55) of ± 30 degrees, and whose width is adapted to the diameter of the screw shaft (55), in particular to the diameter of a threadless part (59) thereof that cooperates with the bore (51).

22. The endoprosthesis of one of claims 1 to 21, **characterized in that** the radius of the articulation face (43) of the directional piece (41) is selected to be so great that the entire directional piece (41), including the connecting part (44), is located inside a cylindrical segment having this radius.

23. The endoprosthesis of claim 7, if need be in combination with one of claims 8 to 22, **characterized in that** in the rotating piece (33), a bore (93) that is concentric with the articulation face (35') about the second axis (37) is provided; and that a cylindrical body (95) is disposed in the bore (93), which body (95) is connected to a screw shaft (55), and the wall between the bore (93) and the articulation face (35') has a slot (51') extending transversely to the second axis (37), through which slot the screw shaft (55) passes; and that the directional piece (41) has an articulation shim (97) and a pressure shim (99), and the articulation shim (97) has a bore for the screw shaft (55) and an articulation face (43') corresponding to the articulation face (35') of the rotating piece (33), and on the opposite side also has a pressure face cooperating with the pressure shim (99); and that the pressure shim (99) is connected to the screw shaft (55), and with a relative rotation between the screw shaft (55) and the cylindrical body (95) and/or the pressure shim (99), the distance between the cylindrical body (95) and the pressure shim (99) can be varied.

24. The endoprosthesis of one of claims 19 to 23, **characterized in that** the rotating piece (33) is fixable on the shaft part (11) with a ring (91) that can be screwed onto or into the shaft part (11).

## Revendications

1. Endoprothèse pour une articulation scapulo-humérale,
avec une partie tige (11) qui présente une partie tige axiale (13) pour implanter dans l'humérus (86) et une tête de tige (17),
avec une surface d'articulation (25, 29, 35, 35') dans la zone de la tête de tige (17),
et une pièce directionnelle (41) qui est articulée sur la tête de tige (17), qui s'étend le long d'un axe directionnel (46), pièce directionnelle qui est pivotante dans toutes les directions par rapport à la partie tige (11) et qui peut être fixée dans une position de pivotement pouvant être sélectionnée par rapport à la partie tige (11),
ainsi qu'une calotte de tête (65) pouvant être reliée à la partie tige (11) par la pièce directionnelle (41),
**caractérisée en ce**
**qu'**une pièce de rotation (33) est placée entre la tête de tige (17) et la pièce directionnelle (41),
qui est rotative autour d'un premier axe (27) par rapport à la partie tige (11),
**que** la pièce directionnelle (41) est positionnée rotative par rapport à la pièce de rotation (33) autour d'un second axe (37),
**que** le second axe (37) est transversal par rapport au premier axe (27) et transversal par rapport à l'axe directionnel (46),
et **que** des surfaces d'articulation ou de contact (25, 29, 35, 35', 43, 43', 43") entre la partie tige (11) et la pièce de rotation (33) et entre la pièce de rotation (33) et la pièce directionnelle (41) ne permettent respectivement qu'un mouvement relatif entre la partie tige (11) et la pièce de rotation (33), ou entre la pièce de rotation (33) et la pièce directionnelle (41) autour du premier axe commun respectif (27) ou du second axe commun respectif (37) comme centre de rotation.

2. Endoprothèse selon la revendication 1, **caractérisée par** au moins un anneau excentrique (63, 64) indépendant de la pièce directionnelle (41) ou par un anneau à trou oblong (87) entre la pièce directionnelle (41) et la calotte de tête (65).

3. Endoprothèse selon la revendication 1 ou 2, **caractérisée en ce que** la pièce directionnelle (41) présente une surface de cône ou de cylindre extérieure (45) dont le centre de rotation est l'axe directionnel (46), surface de cône ou de cylindre (45) sur laquelle la calotte de tête (65), l'anneau excentrique (63, 64) ou l'anneau à trou oblong (87) peut être rapporté.

4. Endoprothèse selon l'une des revendications 1 à 3, **caractérisée en ce que** la direction du premier axe (27) présente une déviation de direction par rapport à la direction de l'axe de col de moins de 60 degrés, de préférence de moins de 50 degrés.

5. Endoprothèse selon l'une des revendications 1 à 4, **caractérisée en ce que** la direction du premier axe (27) est pratiquement parallèle à un plan qui traverse l'axe de la tige (15) et l'axe du col.

6. Endoprothèse selon l'une des revendications 1 à 5, **caractérisée en ce que** la pièce de rotation (33) qui peut tourner autour du premier axe (27) par rapport à la partie tige (11) présente une surface de corps de rotation axiale concave (35) orientée contre la pièce directionnelle (41) avec le second axe (37) comme centre de rotation.

7. Endoprothèse selon l'une des revendications 1 à 5, **caractérisée en ce que** la pièce de rotation (33) qui peut tourner autour du premier axe (27) par rapport à la partie tige (11) présente une surface de corps de rotation axiale convexe (35) orientée contre la pièce directionnelle (41) avec le second axe (37) comme centre de rotation.

8. Endoprothèse selon l'une des revendications 1 à 7, **caractérisée en ce que** le premier axe (27) coupe le second axe (37) en un point central.

9. Endoprothèse selon l'une des revendications 1 à 7, **caractérisée en ce que** les deux axes (27) sont espacés l'un de l'autre.

10. Endoprothèse selon l'une des revendications 1 à 9, **caractérisée en ce que** le premier axe (27) est transversal par rapport à l'axe de col.

11. Endoprothèse selon l'une des revendications 1 à 9, **caractérisée en ce que** le premier axe (27) est parallèle à l'axe du col.

12. Endoprothèse selon l'une des revendications 1 à 11, **caractérisée en ce que** le premier axe (27) coupe l'axe du col ou coïncide avec celui-ci.

13. Endoprothèse selon l'une des revendications 1 à 12, **caractérisée en ce que** les mouvements de rotation autour du premier axe (27) et du second axe (37) peuvent être fixés individuellement.

14. Endoprothèse selon l'une des revendications 1 à 13, **caractérisée en ce que** les mouvements de rotation autour du premier axe (27) et du second axe (37) peuvent être fixés en commun.

15. Endoprothèse selon la revendication 2, éventuellement en relation avec l'une des revendications 3 à 14, pour laquelle un anneau excentrique unique (63) est utilisé, **caractérisée en ce que** la calotte de tête (65) présente une surface de cône intérieure (73) placée excentrique par rapport à l'axe de la calotte (81), surface qui peut être rapportée sur la surface de cône extérieure (71) de l'anneau excentrique (63).

16. Endoprothèse selon la revendication 15, **caractérisée par** une tête de manipulation qui présente une surface de cône intérieure (73) excentrique par rapport à l'axe de la calotte (81), surface qui peut être rapportée sur la surface de cône extérieure (71) de l'anneau excentrique (63) et dont l'excentricité correspond à l'excentricité de la surface de cône intérieure (73) de la calotte de tête et qui est configurée circulaire de telle manière que l'anneau excentrique (63) est visible et réglable par l'ouverture annulaire de la tête de manipulation.

17. Endoprothèse selon la revendication 2, éventuellement en relation avec l'une des revendications 3 à 15, **caractérisée par** un anneau à trou oblong (87) qui présente un trou oblong (89) avec lequel il peut être posé sur une pièce de jonction (44) de la pièce directionnelle (41) et avec un dispositif de blocage (91) avec lequel l'anneau à trou oblong (87) peut être fixé dans n'importe quelle position de déplacement et de rotation par rapport à la pièce directionnelle (41) sur celle-ci.

18. Endoprothèse selon la revendication 17, **caractérisée en ce que** l'évidement (73) qui coopère avec l'anneau à trou oblong (87) est placé dans la calotte de tête (65) de manière concentrique avec l'axe de la calotte (81).

19. Endoprothèse selon l'une des revendications 1 à 16, **caractérisée en ce qu'**il existe un évidement (25) dans la partie tige (11) avec une paroi latérale (25) qui correspond à une surface de rotation autour du premier axe (27) qu'une pièce de rotation (33) avec des contours qui correspondent à la paroi latérale (25) y est positionnée rotative autour du premier axe (27), laquelle pièce de rotation (33) présente une surface d'articulation (35, 35') pour la pièce directionnelle (41) en forme de surface de rotation autour du second axe (37), la pièce directionnelle (41) présente une forme antagoniste (43, 43') correspondante à cette surface d'articulation et une surface de cône axiale (45) pour rapporter une surface de cône intérieure (69) de la calotte de tête (65) ou d'un anneau excentrique (63).

20. Endoprothèse selon la revendication 19, **caractérisée en ce qu'**il est prévu une forure filetée (31) dans la partie tige (11) et la pièce directionnelle (41) présente une forure intérieure (47) dans laquelle la tête (61) d'une vis (53) trouve place, que la pièce de rotation (33) et la pièce directionnelle (41) présentent une forure (39, 51) et que la pièce directionnelle (31) avec la vis (53) peut être vissée par les forures (39, 51) dans la forure filetée (31).

21. Endoprothèse selon la revendication 20, **caractérisée en ce que** la forure (51) est configurée dans la pièce directionnelle (41) pour la tige de vis (55) comme fente qui permet une plage de mouvement de la tige de vis (55) de ± 30° et dont la largeur est adaptée au diamètre de la tige de vis (55) en particulier d'une partie sans filet (59) de celle-ci qui coopère avec la forure (51).

22. Endoprothèse selon l'une des revendications 1 à 21, **caractérisée en ce que** le rayon de la surface d'articulation (43) de la pièce directionnelle (41) est choisi si grand que l'ensemble de la pièce directionnelle (41) y compris la pièce de jonction (44) se trouve à l'intérieur d'une pièce cylindrique avec ce rayon.

23. Endoprothèse selon la revendication 7, éventuellement en relation avec l'une des revendications 8 à 22, **caractérisée en ce qu'**il est prévu dans la pièce de rotation (33) une forure concentrique (93) avec la surface d'articulation (35') autour du second axe (37) et qu'un corps de forme cylindrique (95) est placé dans la forure (93), lequel corps (95) est relié à une tige de vis (55), la paroi entre la forure (93) et la surface d'articulation (35') présente une fente (51') transversale par rapport au second axe (37) par laquelle la tige de vis (55) passe et que la pièce directionnelle (41) présente un disque d'articulation (97) et un disque de pression (99), le disque d'articulation (97) présentant une forure pour la tige de vis (55) et une surface d'articulation (43") correspondante à la surface d'articulation (35') de la pièce de rotation (33) ainsi qu'une surface de pression qui coopère avec le disque de pression (99) sur le côté opposé à cette surface d'articulation et que le disque de pression (99) est en relation avec la tige de vis (55), la distance entre le corps cylindrique (95) et le disque de pression (99) étant variable avec une rotation relative entre la tige de vis (55) et le corps cylindrique (95) et/ou le disque de pression (99).

24. Endoprothèse selon l'une des revendications 19 à 23, **caractérisée en ce que** la pièce de rotation (33) peut être fixée sur la partie tige (11) avec un anneau (91) qui peut être vissé sur ou dans la partie tige (11).
